# FASCICULE DE BREVET EUROPEEN

(11) **EP 0 646 108 B1**
(45) Date de publication et mention de la délivrance du brevet: **14.10.1998**
(21) Numéro de dépôt: 93913126.4
(22) Date de dépôt: 15.06.1993
(51) Int. Cl.: C07C 215/16, A61K 7/13

(54) **2-NITRO-P-PHENYLENEDIAMINES HYDROXYPROPYLEES, LEUR PROCEDE DE PREPARATION ET LEUR UTILISATION EN TEINTURE DES FIBRES KERATINIQUES**
HYDROXYPROPYL-SUBSTITUIERTE 2-NITRO-P-PHENYLENDIAMINE, VERFAHREN ZU IHRER HERSTELLUNG UND IHRE VERWENDUNG ALS FÄRBEMITTEL VON KERATINISCHEN FASERN
HYDROXYPROPYLATED 2-NITRO-P-PHENYLENEDIAMINES, PREPARATION METHOD THEREFOR AND USE THEREOF FOR DYEING KERATIN FIBRES

(30) Priorité: 19.06.1992 FR 9207515
(43) Date de publication de la demande: 05.04.1995
(73) Titulaire: L'OREAL, 75008 Paris (FR)
(72) Inventeur: LAGRANGE, Alain, F-78400 Chatou (FR); JUNINO, Alex, F-93190 Livry-Gargan (FR); GENET, Alain, F-93600 Aulnay-sous-Bois (FR); COTTERET, Jean, F-78480 Verneuil-sur-Seine (FR)
(74) Mandataire: Casalonga, Axel
(86) Numéro de dépôt international: FR9300570
(87) Numéro de publication internationale: WO9400414

(56) Documents cités:
- DE-A- 3 616 720
- FR-A- 1 101 904
- FR-A- 1 454 314
- GB-A- 2 164 656
- GB-A- 2 164 959

## Description

La présente invention concerne de nouveaux colorants nitrés benzéniques du type 2-nitro-p-phénylènediamines hydroxypropylées, destinés à la teinture des fibres kératiniques, et en particulier des cheveux humains.

Dans le domaine de la coloration capillaire, l'utilisation des colorants directs est très répandue car ils présentent certains avantages par rapport aux précurseurs de colorants d'oxydation et, notamment, une diminution des risques potentiels d'allergie et l'absence de sensibilisation du cheveu due au processus oxydatif.

Parmi les colorants directs les plus utilisés figurent les dérivés nitrés benzéniques qui, d'une part, présentent une forte affinité pour le cheveu et qui, d'autre part, grâce à la variété des substituants possibles, permettent de couvrir une large gamme de nuances allant du jaune au bleu en passant par le rouge.

Parmi les colorants nitrés benzéniques bleu à bleu violet utilisés, on peut citer notamment le 1-(β-hydroxyéthyl)amino-4-N,N-bis(β-hydroxyéthyl)amino-2-nitrobenzène décrit dans le brevet français 1 101 904, le 1-(γ-hydroxypropyl)amino-4-N,N-bis(β-hydroxyéthyl)-amino-2-nitrobenzène décrit dans le brevet français 2 570 375 et la demande de brevet GB-A-2 164 656, le 1-(β-hydroxyéthyl)amino-4-(N-méthyl, N-β-hydroxyéthyl)amino-2-nitrobenzène décrit dans le brevet canadien 900490 ou le brevet FR-A-1 454 314, le 1-(β-hydroxyéthyl)amino-4-(N-éthyl, N-β-hydroxyéthyl)amino-2-nitrobenzène décrit dans le brevet EP-0184061, le 1-(β,γ-dihydroxypropyl)amino-4-(N-éthyl, N-β-hydroxyéthyl)amino-2-nitrobenzène décrit dans le brevet allemand 3 616 720 et le 1-(β-hydroxyéthyl)amino-4-(N-β-hydroxyéthyl, N-y-hydroxypropyl)amino-2-nitrobenzène décrit dans la demande de brevet GB-A-2 164 959.

Toutefois, la formulation de ces colorants pose des problèmes du fait de leur résistance au lavage, qui n'est pas satisfaisante.

La demanderesse a donc recherché d'autres colorants nitrés benzéniques bleu à bleu violet présentant une bonne solubilité dans l'eau, dans les mélanges eau/alcool et plus généralement dans les supports de teinture usuels et qui conduisent, sur les cheveux, à des teintures stables au lavage, et aussi à la lumière, aux intempéries, et à la transpiration.

C'est à la suite de ces recherches que la demanderesse a découvert de nouvelles 2-nitro p-phénylènediamines ayant pour formule : 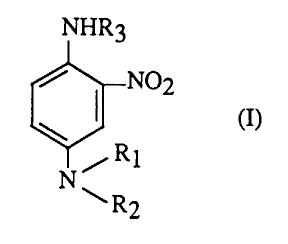 dans laquelle
- R₁ représente un radical alkyle en C₁-C₄,
- R₂ et R₃, indépendamment l'un de l'autre, représentent un radical β-hydroxyéthyle, β-hydroxypropyle, γ-hydroxypropyle ou β,γ-dihydroxypropyle, l'un au moins des radicaux R₂ ou R₃ représentant un radical γ-hydroxypropyle.

Les composés de formule (I) peuvent être utilisés sous forme de base libre ou salifiée par des acides tels que l'acide chlorhydrique, l'acide bromhydrique, l'acide sulfurique etc. ns peuvent donc se trouver sous forme de chlorhydrate, bromhydrate, sulfate etc.

La présente invention a donc pour objet les nouveaux composés de formule (I) ainsi que leurs sels cosmétiquement acceptables.

Dans la formule (I), le radical alkyle en C₁-C₄ représenté par R₁ est un radical méthyle, éthyle, n-propyle, isopropyle, n-butyle, isobutyle ou tert.-butyle.

Les composés préférés de formule (I) sont ceux dans lesquels R₁ désigne un radical méthyle, éthyle ou n-propyle, R₂ désigne un radical β-hydroxyéthyle ou γ-hydroxypropyle et R₃ désigne un radical β-hydroxyéthyle, β-hydroxypropyle ou γ-hydroxypropyle.

Les composés (I) préférés sont notamment choisis parmi les composés suivants: 1-(γ-hydroxypropyl)amino-4-(N-éthyl, N-β-hydroxyéthyl)amino-2-nitrobenzène, 1-(γ-hydroxypropyl)amino-4-(N-méthyl, N-β-hydroxyéthyl)amino-2-nitrobenzène, 1-(y-hydroxypropyl)-amino-4-(N-n-propyl, N-β-hydroxyéthyl)amino-2-nitrobenzène, 1-(γ-hydroxypropyl)amino-4-(N-éthyl, N-γ-hydroxypropyl)amino-2-nitrobenzène, 1-(β-hydroxyéthylamino-4-(N-éthyl, N-γ-hydroxypropyl)amino-2-nitrobenzène et 1-(β-hydroxypropyl)amino-4-(N-méthyl, N-γ-hydroxypropyl)-amino-2-nitro-benzène et les sels cosmétiquement acceptables de ces composés.

Selon un premier mode de réalisation du procédé de préparation des composés (I), on fait réagir en milieu aqueux, un 1,4-bis(hydroxyalkylamino)-2-nitrobenzène de formule : 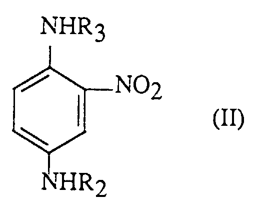 dans laquelle R₂ et R₃ sont identiques ou différents et désignent un radical β-hydroxyéthyle, β-hydroxypropyle, γ-hydroxypropyle ou β,γ-dihydroxypropyle, avec un halogéno alcane en C₁₋C₄ ou un halogéno alcanol en C₁₋C₃ de formule R₁X dans laquelle R₁ est un radical alkyle en C₁₋C₄ et X est un halogène choisi parmi le chlore, le brome et l'iode, en présence de carbonate de calcium, à une température comprise entre la température ambiante et la température de reflux du milieu réactionnel, puis on recueille le composé de formule (I) obtenu et on le purifie éventuellement.

Le composé de formule (II) ci-dessus peut être obtenu en faisant réagir en milieu solvant, au bain-marie bouillant, un 4-fluoro-3-nitro-1-hydroxyalkylaminobenzène de formule: 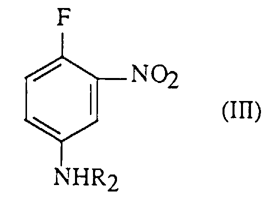 dans laquelle R₂ a la signification indiquée ci-dessus, pour la formule (I), avec une alcanolamine de formule R₃NH₂ dans laquelle R₃ a la signification indiquée ci-dessus pour la formule (I) pour obtenir le composé de formule :

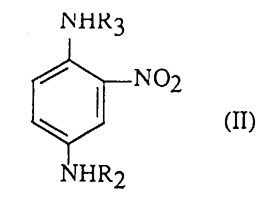

La présente invention a également pour objet les composés nouveaux de formule : 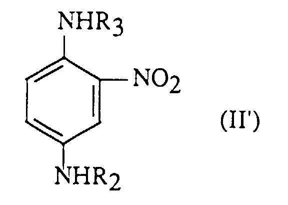 dans laquelle R₂ désigne γ-hydroxypropyle et R₃ désigne β-hydroxyéthyle, β-hydroxypropyle ou γ-hydroxypropyle, ainsi que leurs sels cosmétiquement acceptables.

Les composés de formule (II') ci-dessus sont des colorants violet à rouge pouvant être utilisés comme colorants directs en teinture des fibres kératiniques, et notamment des cheveux humains.

L'invention a donc également pour objet une composition tinctoriale pour la coloration directe des fibres kératiniques, en particulier des cheveux humains, contenant dans un véhicule aqueux, alcoolique ou hydroalcoolique, au moins un composé de formule (I) indiquée ci-dessus ou bien au moins un composé de formule (Il') indiquée ci-dessus, ou l'un de leurs sels cosmétiquement acceptables,

La demanderesse a constaté que lorsqu'on utilise un colorant bleu à bleu violet de formule (I) suivante: 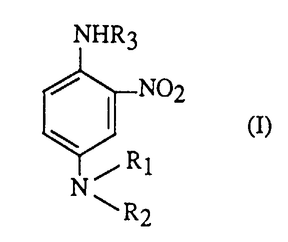 dans laquelle
- R₁ représente un radical alkyle en C₁-C₄, β-hydroxyéthyle, β-hydroxypropyle ou γ-hydroxypropyle;
- R₂ et R₃, indépendamment l'un de l'autre, représentent un radical β-hydroxyéthyle, β-hydroxypropyle, γ-hydroxypropyle ou β,γ-dihydroxypropyle, l'un au moins des radicaux R₁, R₂ ou R₃ représentant un radical γ-hydroxypropyle et les deux autres radicaux ne pouvant désigner simultanément un radical β-hydroxyéthyle,
ou le colorant violet à rouge de formule (II'), en association avec un ou plusieurs colorants jaune ou jaune vert, on obtient, notamment sur des cheveux naturels gris à 90% de blancs ou sur des cheveux gris permanentés, des nuances naturelles plus solides au lavage, à la lumière, aux intempéries et à la transpiration que lorsqu'on utilise les colorants de l'art antérieur.

L'invention a donc aussi pour objet une composition tinctoriale pour la coloration directe des fibres kératiniques, en particulier des cheveux humains, contenant dans un véhicule aqueux, alcoolique ou hydroalcoolique, au moins un composé de formule (I) ci-dessus ou un composé de formule (II'), ou l'un de leurs sels cosmétiquement acceptables, en association avec un ou plusieurs colorants nitrés benzéniques jaune ou jaune vert choisis parmi les composés suivants :
- 1-β-hydroxyéthyloxy-3-méthylamino-4-nitrobenzène,
- 1-(méthylamino)-2-nitro-5-(β,γ-dihydroxypropyl)oxybenzène,
- 1-(β-hydroxyéthylamino)-2-méthoxy-4-nitrobenzène,
- 1-(β-aminoéthylamino)-2-nitro-5-méthoxy-benzène,
- 1,3-di(β-hydroxyéthylamino)-4-nitro-6-chlorobenzène,
- 1-amino-2-nitro-6-méthyl-benzène,
- 1-(β-hydroxyéthylamino)-2-hydroxy-4-nitrobenzène,
- N-(β-hydroxyéthyl)-2-nitro-4-trifluorométhylaniline,
- acide 4-β-hydroxyéthylamino-3-nitro-benzènesulfonique,
- acide 4-éthylamino-3-nitro-benzoïque,
- 4-(β-hydroxyéthyl)amino-3-nitro-chlorobenzène,
- 4-(β-hydroxyéthyl)amino-3-nitro-méthylbenzène,
- 4-(β,γ-dihydroxypropyl)amino-3-nitro-trifluorométhylbenzène,
- 1-β-uréidoéthylamino-4-nitrobenzène,
- O,N-bis(β-hydroxyéthyl)-2-amino-5-nitrophénol,
- 1,3-diamino-4-nitrobenzène,
- 1-hydroxy-2-amino-5-nitrobenzène,
- 1-amino-2-[tris(hydroxyméthyl)méthyl]amino-5-nitro-benzène,
- 1-(β-hydroxyéthyl)amino-2-nitrobenzène,
- 4-(β-hydroxyéthylamino)-3-nitrobenzamide.

Selon l'invention, la concentration en composé de formule (I) ou (II') est comprise entre 0,01 et 10% en poids, et de préférence entre 0,1 et 5% en poids, exprimée en base libre, par rapport au poids total de la composition.

La concentration totale en colorants jaune ou jaune vert est comprise entre 0,05 et 3% en poids, sur la base du poids total de la composition.

On peut bien entendu ajouter aux associations de colorants (I) ou (II') et de colorants jaune ou jaune vert selon l'invention, d'autres colorants nitrés benzéniques, par exemple les colorants rouges choisis parmi les composés suivants :
- 1-hydroxy-3-nitro-4-(γ-hydroxypropylamino)benzène,
- N-(β-hydroxyéthyl)amino-3-nitro-4-aminobenzène,
- 1-amino-3-méthyl-4-(β-hydroxyéthyl)amino-6-nitrobenzène,
- 1-hydroxy-3-nitro-4-N-β-hydroxyéthyl aminobenzène,
- 1,4-diamino-2-nitrobenzène,
- 1-amino-2-nitro-4-méthylaminobenzène,
- N-(β-hydroxyéthyl)-2-nitro-paraphénylènediamine,
- 1-amino-2-nitro-4-(β-hydroxyéthylamino)-5-chlorobenzène,
- 2-nitro-4-amino-diphénylamine,
- 1-amino-3-nitro-6-hydroxybenzène.

On peut également ajouter des colorants nitrés benzéniques orangés choisis parmi les composés suivants :
- -(β-aminoéthyl)amino-2-nitro-4-(β-hydroxyéthyl)oxybenzène,
- 1-(β,γ-dihydroxypropyl)oxy-3-nitro-4-(β-hydroxyéthyl)aminobenzène,
- 1-hydroxy-3-nitro-4-aminobenzène,
- 1-hydroxy-2-amino-4,6-dinitrobenzène,
- 1-méthoxy-3-nitro-4-(β-hydroxyéthylamino)benzène,
- 2-nitro-4'-hydroxydiphénylamine,
- 1-amino-2-nitro-4-hydroxy-5-méthylbenzène.

On peut aussi ajouter d'autres colorants directs tels que des colorants azoïques, des colorants anthraquinoniques, des colorants dérivés du triarylméthane ou des colorants basiques parmi lesquels on peut citer plus particulièrement les colorants connus sous les dénominations "Basic Brown 16", "Basic Yellow 57", "Basic Red 76" et "Basic Blue 99" dans le COLOR INDEX, 3e édition.

La proportion de ces colorants d'addition, nitrés benzéniques rouges ou orangés ou autres colorants directs, peut varier entre 0,05 et 10% en poids de la composition.

La composition tinctoriale selon l'invention peut comprendre, comme véhicule approprié, l'eau et/ou des solvants organiques acceptables sur le plan cosmétique et plus particulièrement des alcools tels que l'alcool éthylique, l'alcool isopropylique, l'alcool benzylique et l'alcool phényléthylique ou des glycols ou éthers de glycols tels que, par exemple, l'éthylèneglycol et ses éthers monométhylique, monoéthylique et monobutylique, le propylèneglycol, le butylèneglycol, le dipropylèneglycol ainsi que les alkyléthers de diéthylèneglycol, comme par exemple le monoéthyléther ou le monobutyléther du diéthylèneglycol, dans des concentrations comprises entre 0,5 et 20% et, de préférence, entre 2 et 10% par rapport au poids total de la composition.

On peut également ajouter à la composition selon l'invention des amides gras tels que les mono- et diéthanolamides des acides dérivés du coprah, de l'acide laurique ou de l'acide oléïque, à des concentrations comprises entre 0,05 et 10% en poids.

On peut aussi ajouter à la composition selon l'invention des agents tensio-actifs anioniques, cationiques, non-ioniques, amphotères ou leurs mélanges. De préférence, les tensio-actifs sont présents dans la composition selon l'invention en une proportion comprise entre 0,1 et 50% en poids et avantageusement, entre 1 et 20% en poids par rapport au poids total de la composition.

Parmi les agents tensio-actifs, on peut citer plus particulièrement les agents tensio-actifs anioniques utilisés seuls ou en mélange tels que, notamment, les sels alcalins, les sels de magnésium, les sels d'ammonium, les sels d'amine ou les sels d'alcanolamine des composés suivants :
- alkylsulfates, alkyléthersulfates, alkylamidesulfates éthoxylés ou non, alkylsulfonates, alkylamide sulfonates, alphaoléfinesulfonates;
- alkylsulfoacétates, alkylphosphates;
- acides gras tels que les acides laurique, myristique, oléïque, ricinoléïque, palmitique, stéarique, les acides d'huile de coprah ou d'huile de coprah hydrogénée, les acides carboxyliques d'éthers polyglycoliques,

les radicaux alkyle de ces composés ayant une chaîne linéaire de 12 à 18 atomes de carbone.

A titre d'agents tensio-actifs cationiques, on peut citer plus particulièrement les sels d'amines grasses, les sels d'ammonium quaternaire tels que les chlorures et bromures d'alkyldiméthylbenzylammonium, d'alkyltriméthylammonium, d'alkyl-diméthylhydroxyéthylammonium, de diméthyldialkylammonium, les sels d'alkylpyridinium, les dérivés d'imidazoline. Les groupements alkyle des dérivés d'ammonium quaternaire précités sont des groupements à chaîne longue ayant, de préférence, entre 12 et 18 atomes de carbone.

Parmi ces composés à caractère cationique on peut également citer les oxydes d'amines.

Parmi les agents tensio-actifs amphotères qui peuvent être utilisés, on peut citer en particulier les alkylamino (mono- et di) propionates, les bétaïnes telles que les alkyl-bétaïnes, les N-alkyl-sulfobétaïnes, les N-alkylaminobétaïnes, le radical alkyle ayant entre 8 et 22 atomes de carbone, les cycloimidiniums tels que les alkylimidazolines.

Parmi les tensio-actifs non ioniques qui peuvent éventuellement être utilisés dans les compositions conformes à l'invention, on peut mentionner les alcools, α-diols, alkylphénols et amides, polyglycérolés, ces composés comportant une chaîne grasse en C₈-C₁₈;
les alcools, alkylphénols et acides gras, polyéthoxylés, ces composés comportant une chaîne grasse en C₈ à C₁₈;
les condensats d'oxyde d'éthylène et de propylène sur des alcools gras; les amides gras polyéthoxylés, contenant au moins 5 moles d'oxyde d'éthylène;
les amines grasses polyéthoxylées.

Les produits épaississants, que l'on peut ajouter dans la composition selon l'invention, peuvent avantageusement être pris dans le groupe formé par l'alginate de sodium, la gomme arabique, la gomme de guar, la gomme de caroube, la gomme de xanthane, les dérivés de la cellulose tels que la méthylcellulose, l'hydroxyéthylcellulose, l'hydroxypropylméthylcellulose, le sel de sodium de la carboxyméthylcellulose et les polymères d'acide acrylique.

On peut également utiliser des agents épaississants minéraux tels que la bentonite. Ces épaississants sont utilisés seuls ou en mélange, et, de préférence, sont présents en une proportion comprise entre 0,2 et 5% en poids par rapport au poids total de la composition et, avantageusement, entre 0,5 et 3% en poids.

La composition tinctoriale selon l'invention peut être formulée à pH acide, neutre ou alcalin, le pH pouvant varier de 4 à 10,5 et, de préférence, de 5 à 10. Parmi les agents d'alcalinisation qui peuvent être utilisés, on peut mentionner les alcanolamines, les hydroxydes et les carbonates alcalins ou d'ammonium. Parmi les agents d'acidification qui peuvent être utilisés, on peut mentionner l'acide lactique, l'acide acétique, l'acide tartrique, l'acide phosphorique, l'acide chlorhydrique et l'acide citrique.

La composition tinctoriale selon l'invention peut contenir en outre divers adjuvants usuels tels que des agents anti-oxydants, des parfums, des agents séquestrants, des produits filmogènes et des agents de traitement, des agents dispersants, des agents de conditionnement du cheveu, des agents conservateurs, des agents opacifiants, ainsi que tout autre adjuvant utilisé habituellement en cosmétique.

La composition tinctoriale selon l'invention peut se présenter sous les diverses formes usuelles pour la teinture des cheveux, telles que des liquides épaissis ou gélifiés, des crèmes, des mousses en aérosols ou sous toutes autres formes appropriées pour réaliser une teinture de fibres kératiniques.

La présente invention a également pour objet un procédé de teinture des fibres kératiniques, et notamment des cheveux humains, consistant à laisser agir la composition tinctoriale ci-dessus définie sur les fibres kératiniques séches ou humides. On peut utiliser la composition selon l'invention en tant que lotion non rincée, c'est-à-dire qu'on applique la composition selon l'invention sur les fibres kératiniques, puis on sèche sans rinçage intermédiaire. Dans les autres modes d'application, on applique la composition tinctoriale selon l'invention sur les fibres kératiniques pendant un temps de pose variant entre 3 et 60 minutes, de préférence entre 5 et 45 minutes, on rince, éventuellement on lave et on rince à nouveau, puis on sèche.

Pour mieux faire comprendre l'objet de l'invention, on va en décrire maintenant, à titre d'exemples purement illustratifs et non limitatifs, plusieurs modes de mise en oeuvre.

### EXEMPLES DE PREPARATION

### EXEMPLE 1

### Préparation du chlorhydrate de 1-(γ-hydroxypropyl)amino-4-(N-méthyl, N-β-hydroxyéthyl)amino-2-nitrobenzène, dénommé selon la nomenclature IUPAC 3-{4-[(2-hydroxyéthyl)-méthylamino]-2-nitro-phénylamino}-propan-1-ol.

On chauffe au bain d'eau chaude (50°C) la suspension de 51,0 g (0,2 mole) de 1-(γ-hydroxypropylamino)-4-(β-hydroxyéthyl)amino-2-nitrobenzène et de 30 g de carbonate de calcium dans 80 ml d'eau. En une heure, on ajoute goutte à goutte 18,7 ml (0,3 mole) d'iodure de méthyle et on continue le chauffage 7 heures en suivant la réaction en chromatographie sur couche mince (gel de silice; éluant : acétate d'éthyle).

On filtre à chaud et on refroidit : l'huile qui précipite est extraite à l'acétate d'éthyle.

La phase acétate d'éthyle est séchée sur sulfate de sodium, filtrée et évaporée à sec sous pression réduite.

L'huile obtenue est purifiée par passage sur colonne moyenne pression (gel de silice; gradient d'acétate d'éthyle et d'heptane). Après évaporation à sec du solvant, la base libre est dissoute dans 150 ml d'éthanol absolu et on ajoute 25 ml d'une solution d'acide chlorhydrique environ 7N dans l'éthanol absolu.

Le chlorhydrate du composé attendu précipite en cristaux jaunes qui sont essorés, lavés à l'éther éthylique et séchés sur potasse sous vide.

On obtient 22,1 g de chlorhydrate qui fond avec décomposition à 189-192°C et dont l'analyse élémentaire calculée pour C₁₂H₂₀N₃O₄Cl est :

| | C% | H% | N% | O% | Cl% |
|---|---|---|---|---|---|
| Calculé | 47,14 | 6,59 | 13,74 | 20,93 | 11,59 |
| Trouvé | 47,24 | 6,71 | 13,59 | 21,20 | 11,66 |

### EXEMPLE 2

### Préparation du chlorhydrate de 1-(γ-hydroxypropyl)amino-4-(N-éthyl,N-β-hydroxyéthyl)amino-2-nitrobenzène, dénommé selon la nomenclature IUPAC 3-{4-[éthyl-(2-hydroxyéthyl)-amino]-2-nitro-phénylamino}-propan-1-ol.

Ce composé est préparé et purifié selon le mode opératoire décrit pour l'exemple précédent.

A partir de 51,0 g (0.2 mole) de 1-(y-hydroxypropyl)amino)-4-(β-hydroxyéthyl)amino-2-nitrobenzène et de 1-iodo-éthane, on obtient des cristaux jaunes de chlorhydrate (33,1 g) qui fondent avec décomposition à 168-170°C et dont l'analyse élémentaire calculée pour C₁₃H₂₂N₃O₄Cl est :

| | C% | H% | N% | O% | Cl% |
|---|---|---|---|---|---|
| Calculé | 48,83 | 6,93 | 13,14 | 20,01 | 11,09 |
| Trouvé | 49,05 | 6,99 | 12,92 | 20,10 | 10,94 |

### EXEMPLE 3

### Préparation du chlorhydrate de 1-(γ-hydroxypropyl)amino-4-(N-n-propyl, N-β-hydroxyéthyl)amino-2-nitrobenzène, dénommé selon la nomenclature IUPAC 3-{4-[(2-hydroxyéthyl)-propylamino]-2-nitrophénylamino}-propan-1-ol.

Ce composé est préparé et purifié selon le mode opératoire décrit pour l'exemple 1.

A partir de 51,0 g (0,2 mole) de 1-(γ-hydroxypropyl)amino-4-(β-hydroxyéthyl)amio-2-nitrobenzène et de 1-bromo-propane, on obtient des cristaux jaunes de chlorhydrate (43,7g) qui fondent avec décomposition à 140-142°C et dont l'analyse élémentaire calculée pour C₁₄H₂₄N₃O₄Cl est :

| | C% | H% | N% | O% | Cl% |
|---|---|---|---|---|---|
| Calculé | 50,37 | 7,25 | 12,59 | 19,17 | 10,62 |
| Trouvé | 50,28 | 7,32 | 12,62 | 19,41 | 10,45 |

### EXEMPLE 4

### Préparation du chlorhydrate de 1-(γ-hydroxypropl)amino-4-(N-β-hydroxyéthyl,N-γ-hydroxypropyl)amino-2-nitrobenzène, dénommé selon la nomenclature IUPAC 3-{4-[(2-hydroxyéthyl)-(3-hydroxypropyl)-amino]-2-nitro-phénylamino}-propan-1-ol.

Ce composé est préparé et purifié selon le mode opératoire décrit pour l'exemple 1.

A partir de 33,2 g (0,13 mole) de 1-(y-hydroxypropyl)amino-4-(β-hydroxyéthyl)amino-2-nitrobenzène et de 3-chloro-propan-1-ol, on obtient des cristaux jaunes de chlorhydrate (16,3g) qui fondent avec décomposition à 100-102°C et dont l'analyse élémentaire calculée pour C₁₄H₂₄N₃O₄Cl + 1/2 H₂O est:

| | C% | H% | N% | O% | Cl% |
|---|---|---|---|---|---|
| Calculé | 46,86 | 7,02 | 11,71 | 24,52 | 9,88 |
| Trouvé | 46,93 | 6,99 | 11,74 | 23,96 | 10,11 |

### EXEMPLE 5

### Préparation du chlorhydrate de 1-(γ-hydroxypropyl)amino-4-N,N-bis(γ-hydroxypropyl)-amino-2-nitrobenzène, dénommé selon la nomenclature IUPAC 3-{4-[bis-(3-hydroxypropyl)-amino]-2-nitrophénylamino}-propan-1-ol.

### 1) 1ère étape :

### Préparation du 4-fluoro-3-nitro-1-N,N'-bis(γ-hydroxypropyl)aminobenzène

On chauffe pendant 20 heures au bain-marie bouillant, la suspension de 156,1 g (1 mole) de 4-fluoro-3-nitro-aniline, de 236,3 g de 3-chloro-propan-1-ol et de 200 g de carbonate de calcium dans 500 ml d'eau.

Le mélange réactionnel est filtré chaud, refroidi et extrait à l'acétate d'éthyle.

La phase acétate d'éthyle est séchée sur sufate de sodium, filtrée et évaporée à sec sous pression réduite.

Le composé cristallisé obtenu est purifié par passage sur colonne moyenne pression (gel de silice; gradient d'acétate d'éthyle et d'heptane).

Après évaporation à sec du solvant et recristallisation de l'acétate d'isopropyle bouillant, on obtient 84 g de 4-fluoro-3-nitro-1-N,N-bis(y-hydroxypropyl)aminobenzène dont le point de fusion est 69°C et dont l'analyse élémentaire calculée pour C₁₂H₁₇N₂O₄F est :

| | C% | H% | N% | F% |
|---|---|---|---|---|
| Calculé | 52,94 | 6,29 | 10,29 | 6,98 |
| Trouvé | 53,02 | 6,46 | 10,29 | 7,01 |

### 2) 2e étape :

### Préparation du chlorhydrate de 1-(γ-hydroxypropyl)amino-4-N,N-bis(γ-hydroxypropyl)amino-2-nitrobenzène

On chauffe pendant 4 heures au bain-marie bouillant, une solution de 54,4 g (0,2 mole) du 4-fluoro-3-nitro-1-N,N-bis(γ-hydroxypropyl)aminobenzène obtenu à la première étape et de 30 g de 3-aminopropan-1-ol dans 50 ml de dioxane.

Le milieu réactionnel est versé dans 400 ml d'eau glacée, neutralisé avec une solution d'acide chlorhydrique à 36% et extrait à l'acétate d'éthyle.

La phase acétate d'éthyle est séchée sur sulfate de sodium, filtrée et évaporée à sec sous pression réduite.

L'huile obtenue est purifiée par passage sur colonne moyenne pression (gel de silice; gradient d'acétate d'éthyle et d'heptane).

Après évaporation à sec du solvant, la base libre est dissoute dans 250 ml d'éthanol absolu et on ajoute 15 ml d'une solution d'acide chlorhydrique environ 7N dans l'éthanol absolu.

Le chlorhydrate du composé attendu précipite en cristaux jaunes qui sont essorés, lavés à l'éther éthylique et séchés sur potasse sous vide.

On obtient 16 g de chlorhydrate qui fond avec décomposition à 155-157°C et dont l'analyse élémentaire calculée pour C₁₅H₂₆N₃O₅Cl est :

| | C% | H% | N% | O% | Cl% |
|---|---|---|---|---|---|
| Calculé | 49,52 | 7,20 | 11,55 | 21,99 | 9,74 |
| Trouvé | 49,51 | 7,25 | 11,43 | 22,14 | 9,84 |

### EXEMPLE 6

### Préparation du chlorhydrate de 1-(β-hydroxyéthyl)amino-4-N,N-bis(γ-hydroxypropyl)amino-2-nitrobenzène, dénommé selon la nomenclature IUPAC 3-[[4-(2-hydroxyéthylamino)-3-nitrophényl]-(3-hydroxypropyl)-amino]-propan-1-ol.

Ce composé est préparé et purifié selon le mode opératoire décrit pour l'exemple 5 (étape 1).

A partir de 54,4 g de 4-fluoro-3-nitro-1-N,N-bis(y-hydroxypropyl)aminobenzène obtenu à la première étape de l'exemple 5 et de 29,2 g d'éthanolamine, on obtient des cristaux jaunes de chlorhydrate (22,4 g) qui fondent avec décomposition à 170-172°C et dont l'analyse élémentaire calculée pour C₁₄H₂₄N₃O₅Cl est :

| | C% | H% | N% | O% | Cl% |
|---|---|---|---|---|---|
| Calculé | 48,07 | 6,92 | 12,01 | 22,87 | 10,13 |
| Trouvé | 48,06 | 7,01 | 11,92 | 22,83 | 10,04 |

### EXEMPLE 7

### Préparation du chlorhydrate de 1-(γ-hydroxypropyl)amino-4- (N-éthyl, N-γ-hydroxypropyl)-amino-2-nitrobenzène, dénommé selon la nomenclature IUPAC 3-{4-[éthyl-(3-hydroxypropyl)-amino]-2-nitro-phénylamino)-propan-1-ol.

### 1) 1ère étape :

### Préparation du 1-(γ-hydroxypropyl)amino-3-nitro-4-(γ-hydroxypropyl)aminobenzène

On chauffe pendant 3 heures au bain-marie bouillant, une solution de 15,0 g (0,07 mole) de 4-fluoro-3-nitro-1-(γ-hydroxypropyl)aminobenzène dans 30 ml de 3-amino-propan-1-ol et 5 ml de dioxane.

Le milieu réactionnel est versé dans 250 ml d'eau glacée, neutralisé avec une solution d'acide chlorhydrique à 36% et extrait à l'acétate d'éthyle.

La phase acétate d'éthyle est séchée sur sulfate de sodium, filtrée et évaporée à sec sous pression réduite.

L'huile obtenue est purifiée par passage sur colonne moyenne pression (gel de silice; gradient d'acétate d'éthyle et d'heptane).

Après évaporation à sec du solvant, on obtient 10 g d'huile violette de 1-(γ-hydroxypropyl)amino-3-nitro-4-(γ-hydroxypropyl)amino benzène dont l'analyse élémentaire calculée pour C₁₂H₁₉N₃O₄ est :

| | C% | H% | N% | O% |
|---|---|---|---|---|
| Calculé | 53,52 | 7,11 | 15,60 | 23,76 |
| Trouvé | 53,96 | 7,23 | 15,46 | 24,07 |

### 2) 2e étape :

### Préparation du chlorhydrate de 1-(γ-hydroxypropyl)amino-4-(N-éthyl, N-γ-hydroxypropyl)amino-2-nitrobenzène

Ce composé est préparé et purifié selon le mode opératoire décrit pour l'exemple 1.

A partir de 9,3 g (0,034 mole) de 1-(y-hydroxypropyl)amino-3-nitro-4-(γ-hydroxypropyl)aminobenzène préparé ci-desssus (étape 1) et d'iodure d'éthyle, on obtient des cristaux jaunes de chlorhydrate de 1-(y-hydroxypropyl)amino-4-(N-éthyl, N-γ-hydroxypropyl)amino-2-nitrobenzène (5,0 g) qui fondent avec décomposition à 130-132°C et dont l'analyse élémentaire calculée pour C₁₄H₂₄N₃O₄Cl est :

| | C% | H% | N% | O% | Cl% |
|---|---|---|---|---|---|
| Calculé | 50,37 | 7,25 | 12,59 | 19,17 | 10,62 |
| Trouvé | 50,39 | 7,18 | 12,72 | 19,50 | 10,64 |

### EXEMPLE 8

### Préparation du chlorhydrate de 1-(β-hydroxyéthyl)amino-4-(N-éthyl, N-γ-hydroxypropyl)amino-2-nitrobenzène, dénommé selon la nomenclature IUPAC 3-{éthyl-[4-(2-hydroxyéthylamino)-3-nitrophényl]-amino}-propan-1-ol.

### 1) 1ère étape :

### Préparation du 1-(γ-hydroxypropyl)amino-3-nitro-4-(β-hydroxyéthyl)aminobenzène

On suit le mode opératoire décrit pour l'exemple 7 (1ère étape).

A partir de 15,0 g (0,07 mole) de 4-fluoro-3-nitro-1-(γ-hydroxypropyl)aminobenzène dans 30 ml d'éthanolamine et 5 ml de dioxane, on obtient 11 g d'huile violette de 1-(y-hydroxypropyl)amino-3-nitro4-(β-hydroxyéthyl)aminobenzène dont l'analyse élémentaire calculée pour C₁₁H₁₇N₃O₄ est :

| | C% | H% | N% | O% |
|---|---|---|---|---|
| Calculé | 51,76 | 6,71 | 16,46 | 25,07 |
| Trouvé | 52,28 | 7,15 | 16,45 | 25,47 |

### 2) 2e étape :

### Préparation du chlorhydrate de 1-(β-hydroxyéthyl)amino-4-(N-éthyl, N-γ-hydroxypropyl)amino-2-nitrobenzène

Ce composé est préparé et purifié selon le mode opératoire décrit pour l'exemple 1.

A partir de 11,0 g (0,047 mole) de 1-γ-hydroxypropylamino-3-nitro-4-(β-hydroxyéthyl)aminobenzène préparé ci-desssus (étape 1) et d'iodure d'éthyle, on obtient des cristaux jaunes de chlorhydrate de 1-(β-hydroxyéthyl)amino-4-(N-éthyl, N-γ-hydroxypropyl)amino-2-nitrobenzène (6,9 g) qui fondent avec décomposition à 168-170°C et dont l'analyse élémentaire calculée pour C₁₃H₂₂N₃O₄Cl est :

| | C% | H% | N% | O% | Cl% |
|---|---|---|---|---|---|
| Calculé | 48,83 | 6,93 | 13,14 | 20,01 | 11,09 |
| Trouvé | 48,94 | 7,06 | 12,94 | 20,18 | 10,84 |

### EXEMPLE 9

### Préparation du chlorhydrate de 1-(β-hydroxypropyl)amino-4-(N-méthyl, N-γ-hydroxypropyl)amino-2-nitrobenzène, dénommé selon la nomenclature IUPAC 3-{[4-(2-hydroxypropylamino)-3-nitro-phényl]-méthyl-amino}-propan-1-ol.-

### 1) 1ère étape :

### Préparation du 1-(γ-hydroxypropyl)amino-3-nitro-4-(β-hydroxypropyl)aminobenzène

On chauffe 2 heures au bain-marie bouillant une solution de 15,0 g (0,07 mole) de 4-fluoro-3-nitro-1-(γ-hydroxypropyl)aminobenzène dans 30 ml de 1-amino-propan-2-ol et 5 ml de dioxane.

Le milieu réactionnel est versé dans 250 ml d'eau glacée et neutralisé avec une solution d'acide chlorhydrique à 36%.

Le précipité cristallisé rouge est essoré, lavé à l'eau, recristallisé dans l'alcool à 96° bouillant et séché sous vide.

On obtient 17 g de 1-(γ-hydroxypropyl)amino-3-nitro-4-**(β**-hydroxypropyl)aminobenzène dont le point de fusion est 119°C et dont l'analyse élémentaire calculée pour C₁₂H₁₉N₃O₄ est :

| | C% | H% | N% | O% |
|---|---|---|---|---|
| Calculé | 53,52 | 7,11 | 15,60 | 23,76 |
| Trouvé | 53,63 | 7,13 | 15,61 | 24,02 |

### 2) 2e étape:

### Préparation du chlorhydrate de 1-(β-hydroxypropyl)amino-4-(N-méthyl, N-γ-hyroxypropyl)amino-2-nitrobenzène

Ce composé est préparé et purifié selon le mode opératoire décrit pour l'exemple 1.

A partir de 16,5 g (0,061 mole) de 1-(γ-hydroxypropyl)amino-3-nitro-4-(β-hydroxypropyl)aminobenzène préparé ci-desssus (étape 1) et d'iodure de méthyle, on obtient des cristaux jaunes de chlorhydrate de 1-(β-hydroxypropyl)amino-4-(N-méthyl, N-γ-hydroxypropyl)amino-2-nitrobenzène (5,9 g) qui fondent avec décomposition à 158-160°C et dont l'analyse élémentaire calculée pour C₁₃H₂₂N₃O₄Cl + 1/4 H₂O est :

| | C% | H% | N% | O% | Cl% |
|---|---|---|---|---|---|
| Calculé | 48,15 | 6,99 | 12,96 | 20,97 | 10,93 |
| Trouvé | 48,23 | 6,94 | 13,04 | 20,89 | 10,92 |

### EXEMPLES DE TEINTURE

### EXEMPLE A

On prépare la composition tinctoriale suivante :
- Chlorhydrate de 1-(γ-hydroxypropyl)amino-4-(N-méthyl, N-β-hydroxyéthyl)amino-2-nitrobenzène 0,92 g
- Monométhyléther de propylène glycol 10 g
- Diéthanolamide d'acides gras de coprah 2 g
- Nonylphénol oxyéthyléné à 9 moles d'oxyde d'éthylène 8 g
- 2-amino 2-méthyl 1-propanol q.s. pH 9
- Eau qsp 100 g

On applique la composition ci-dessus sur des cheveux gris naturels à 90% de blancs. On laisse poser 30 minutes à température ambiante. Après rinçage et séchage, les cheveux sont teints dans une couleur violine cendré.

### EXEMPLE B

- Chlorhydrate de 1-(γ-hydroxypropyl)amino-4-(N-éthyl, N-β-hydroxyéthyl)amino-2-nitrobenzène 0,96 g
- Monométhyléther de propylèneglycol 10 g
- Diéthanolamide d'acides gras de coprah 2 g
- Nonylphénol oxyéthyléné à 9 moles d'oxyde d'éthylène 8 g
- 2-amino 2-méthyl 1-propanol q.s. pH 9
- Eau qsp 100 g

On applique la composition ci-dessus sur des cheveux gris naturels à 90% de blancs. On laisse poser 30 minutes à température ambiante. Après rinçage et séchage, les cheveux sont teints dans une couleur cendrée.

### EXEMPLE C

On prépare la composition tinctoriale suivante :
- Chlorhydrate de 1-(γ-hydroxypropyl)amino-4-(N-éthyl, N-γ-hydroxypropyl)amino-2-nitrobenzène 1 g
- Monométhyléther de propylène glycol 10 g
- Diéthanolamide d'acides gras de coprah 2 g
- Nonylphénol oxyéthyléné à 9 moles d'oxyde d'éthylène 8 g
- 2-amino 2-méthyl 1-propanol q.s. pH 9
- Eau qsp 100 g

On applique la composition tinctoriale ci-dessus sur des cheveux gris à 90% de blancs ayant subi un traitement de permanente. Après 30 minutes de pose à température ambiante, rinçage et séchage, les cheveux sont teints dans une couleur cendrée.

### EXEMPLE D

On prépare la composition tinctoriale suivante :
- Chlorhydrate de 1-(β-hydroxyéthyl)amino-4-(N-éthyl, N-γ-hydroxypropyl)amino-2-nitrobenzène 0,96 g
- Monométhyléther de propylène glycol 10 g
- Diéthanolamide d'acides gras de coprah 2 g
- Nonylphénol oxyéthyléné à 9 moles d'oxyde d'éthylène 8 g
- 2-amino 2-méthyl 1-propanol q.s. pH 9
- Eau qsp 100 g

On applique la composition tinctoriale ci-dessus sur des cheveux gris à 90% de blancs, permanentés. Après 30 minutes de pose à température ambiante, rinçage et séchage, les cheveux sont teints dans une couleur violine légèrement cendré.

### EXEMPLE E

On prépare la composition tinctoriale suivante :
- Chlorhydrate de 1-(γ-hydroxypropyl)amino-4-(N-éthyl, N-β-hydroxyéthyl)amino-2-nitrobenzène 0,5 g
- 1-β-hydroxyéthyloxy-3-méthylamino-4-nitrobenzène 0,07 g
- Nonylphénol oxyéthyléné à 9 moles d'oxyde d'éthylène 8 g
- Diéthanolamide d'acides gras de coprah 2 g
- Ethylglycol 10 g
- Triéthanolamine q.s. pH 9
- Eau q.s.p. 100 g

On applique cette composition sur des cheveux gris à 90% de blancs, permanentés. Après 20 minutes de pose à température ambiante, rinçage et séchage, les cheveux sont teints en blond clair cendré.

## Revendications

1. 2-nitro-p-phénylènediamine hydroxypropylée ayant pour formule: 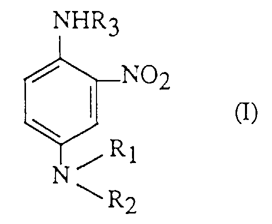 dans laquelle:
- R₁ représente un radical alkyle en C₁-C₄,
- R₂ et R₃, indépendamment l'un de l'autre, représentent un radical β-hydroxyéthyle, β-hydroxypropyle, γ-hydroxypropyle, ou β,γ-dihydroxypropyle, sous réserve que l'un au moins des radicaux R₂ ou R₃ représente un radical γ-hydroxypropyle,
et sels cosmétiquement acceptables de ce composé.

2. Composé selon la revendication 1, caractérisé par le fait que le groupe alkyle en C₁-C₄ représenté par R₁ est un radical méthyle, éthyle ou n-propyle.

3. Composé selon la revendication 1 ou 2, caractérisé par le fait qu'il est choisi parmi les composés suivants: 1-(γ-hydroxypropyl) amino-4-(N-éthyl, N-β-hydroxyéthyl)amino-2-nitrobenzène, 1-(γ-hydroxypropyl)amino-4-(N-méthyl, N-β-hydroxyéthyl)amino-2-nitro benzène, 1-(γ-hydroxypropyl)-amino-4-(N-n-propyl,N-β-hydroxyéthyl) amino-2-nitrobenzène, 1-(γ-hydroxypropyl)amino-4-(N-éthyl,N-γ-hydroxypropyl)-amino-2-nitrobenzène, 1-(β-hydroxyéthyl-amino-4-(N-éthyl,N-γ-hydroxypropyl)amino-2-nitrobenzène et 1-(β-hydroxypropyl)amino-4-(N-méthyl,N-γ-hydroxypropyl)-amino-2-nitrobenzène et les sels cosmétiquement acceptables de ces composés.

4. Composé de formule: 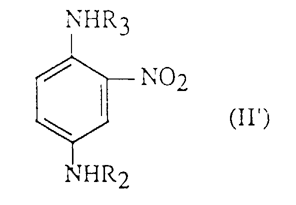 dans laquelle le radical R₂ est le radical γ-hydroxypropyle et le radical R₃ est le radical β-hydroxyéthyle, β-hydroxypropyle ou γ-hydroxypropyle, et ses sels cosmétiquement acceptables.

5. Composition tinctoriale pour la coloration directe de fibres kératiniques, et en particulier des cheveux humains, caractérisée par le fait qu'elle contient dans un véhicule aqueux, alcoolique ou hydroalcoolique, au moins un composé de formule (I) selon l'une quelconque des revendications 1 à 3 ou l'un de ses sels cosmétiquement acceptables .

6. Composition tinctoriale selon la revendication 5, caractérisée par le fait qu'elle contient un composé de formule (I) choisi parmi les composés suivants : 1-(γ-hydroxypropyl)amino-4-(N-éthyl,N-β-hydroxyéthyl) amino-2-nitrobenzène, 1-(γ-hydroxypropyl)amino-4-(N-méthyl,N-β-hydroxyéthyl)amino-2-nitrobenzène, 1-(γ-hydroxypropyl)amino-4-(N-n-propyl, N-β-hydroxyéthyl)amino-2-nitrobenzène, 1-(γ-hydroxypropyl)amino-4-(N-éthyl,N-γ-hydroxypropyl)amino-2-nitrobenzène, 1-(β-hydroxyéthylamino-4-(N-éthyl,N-γ-hydroxypropyl) amino-2-nitrobenzène et 1-(β-hydroxypropyl)amino-4-(N-méthyl,N-γ-hydroxypropyl)amino-2-nitrobenzène et les sels cosmétiquement acceptables de ces composés.

7. Composition tinctoriale pour la coloration directe des fibres kératiniques, en particulier des cheveux humains, caractérisée par le fait qu'elle contient dans un véhicule aqueux, alcoolique ou hydroalcoolique, au moins un composé de formule (Il') selon la revendication 4, ou l'un de ses sels cosmétiquement acceptables.

8. Composition tinctoriale pour la coloration directe de fibres kératiniques, et en particulier de cheveux humains, caractérisée par le fait qu'elle contient, dans un véhicule aqueux, alcoolique ou hydroalcoolique, au moins un composé de formule : 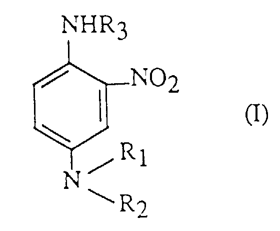 dans laquelle:
- R₁ représente un radical alkyle en C₁-C₄, β-hydroxyéthyle, β-hydroxypropyle, γ-hydroxypropyle,
- R₂ et R₃, indépendamment l'un de l'autre, représentent un radical β-hydroxyéthyle, β-hydroxypropyle, γ-hydroxypropyle, ou β,γ-dihydroxypropyle, sous réserve que l'un au moins des radicaux R₁, R₂ ou R₃ représente un radical γ-hydroxypropyle, et que les deux autres radicaux ne puissent désigner simultanément un radical β-hydroxyéthyle ou (II') selon la revendication 4, ou l'un de leurs sels cosmétiquement acceptables, en association avec au moins un colorant nitré benzénique jaune ou jaune vert choisi parmi les composés suivants:
1-β-hydroxyéthyloxy-3-méthylamino-4-nitrobenzène, 1-(méthylamino)-2-nitro-5-(β,γ-dihydroxypropyl)oxybenzène, 1-(β-hydroxyéthylamino)-2-méthoxy-4-nitrobenzène, 1-(β-aminoéthylamino)-2-nitro-5-méthoxybenzène, 1,3-di(β-hydroxyéthylamino)-4-nitro-6-chlorobenzène, l-amino-2-nitro-6-méthyl-benzène, 1-(β-hydroxyéthylamino)-2-hydroxy-4-nitrobenzène,N-(β-hydroxyéthyl)-2-nitro-4-trifluorométhylaniline, acide 4-β-hydroxyéthylamino-3-nitrobenzène sulfonique, acide 4-éthylamino-3 -nitro-benzoïque,4-(β-hydroxyéthyl)amino-3-nitrochlorobenzène, 4-(β-hydroxyéthyl)amino-3-nitro-méthylbenzène, 4-(β,γ-dihydroxypropyl)amino-3-nitro-trifluorométhylbenzène, 1-β-uréidoéthylamino-4-nitrobenzène, O,N-bis(β-hydroxyéthyl)-2-amino-5-nitrophénol, 1-hydroxy-2-amino-5-nitrobenzène, 1-amino-2-[tris(hydroxyméthyl)-méthyl]amino-5-nitrobenzène, 1-(β-hydroxyéthyl) amino-2-nitrobenzène et 4-(β-hydroxyéthylamino)-3-nitrobenzamide.

9. Composition tinctoriale selon l'une quelconque des revendications 5 à 8, caractérisée par le fait qu'elle contient des colorants nitrés benzéniques rouges choisis parmi les composés suivants: 1 -hydroxy-3-nitro-4-(γ-hydroxypropylamino)benzène, N-(β-hydroxyéthyl)amino-3-nitro-4-aminobenzène, 1-amino-3-méthyl-4-(β-hydroxyéthyl)amino-6-nitro-benzène,1-hydroxy-3-nitro-4-N-(β-hydroxyéthyl)amino-benzène, 1,4-diamino-2-nitrobenzène, 1-amino-2-nitro-4-méthylaminobenzène,N-(β-hydroxyéthyl)-2-nitro-paraphénylènediamine, 1-amino-2-nitro-4-(β-hydroxyéthylamino) 5-chlorobenzène, 2-nitro-4-amino-diphénylamine et 1-amino-3-nitro-6-hydroxybenzène.

10. Composition tinctoriale selon l'une quelconque des revendications 5 à 8, caractérisée par le fait qu'elle contient des colorants nitrés benzéniques orangés choisis parmi les composés suivants: 1-β-aminoéthyl)amino-2-nitro-4-(β-hydroxyéthyl)oxybenzène, 1-(β,γ-dihydroxypropyl)oxy-3-nitro-4-(β-hydroxyéthyl)aminobenzène, 1-hydroxy-3-nitro-4-aminobenzène,1-hydroxy-2-amino-4,6-dinitrobenzène, 1-méthoxy-3-nitro-4-(β-hydroxyéthylamino) benzène, 2-nitro-4'-hydroxy-diphénylamine et 1-amino-2-nitro-4-hydroxy-5-méthylbenzène.

11. Composition tinctoriale selon l'une quelconque des revendications 5 à 10, caractérisée par le fait qu'elle contient d'autres colorants directs choisis parmi les colorants azoïques, anthraquinoniques, les dérivés du triarylméthane et les colorants basiques.

12. Composition tinctoriale selon l'une quelconque des revendications 5 à 11, caractérisée par le fait qu'elle contient 0,01 à 10% en poids, et de préférence 0,1 à 5% en poids, exprimé en base libre, de composé de formule (I) ou (II').

13. Composition tinctoriale selon la revendication 8, caractérisée par le fait qu'elle contient 0,05 à 3% en poids de colorants nitrés benzéniques jaune ou jaune vert.

14. Composition tinctoriale selon l'une quelconque des revendications 5 à 13, caractérisée par le fait qu'elle contient en outre 0,05 à 10% en poids d'autres colorants directs.

15. Composition tinctoriale selon l'une quelconque des revendications 5 à 14, caractérisée par le fait qu'elle contient des solvants organiques choisis parmi les alcools, glycols et éthers de glycols, en des concentrations comprises entre 0,5 et 20% en poids, et de préférence entre 2 et 10% en poids par rapport au poids total de la composition.

16. Composition tinctoriale selon l'une quelconque des revendications 5 à 15, caractérisée par le fait qu'elle contient au moins un adjuvant choisi parmi les amides gras en des concentrations comprises entre 0,05 et 10% en poids, les agents tensio-actifs anioniques, cationiques, non ioniques, amphotères ou leurs mélanges, en des concentrations comprises entre 0,1 et 50% en poids, les épaississants en des concentrations comprises entre 0,2 et 5% en poids, les agents anti-oxydants, les parfums, les agents séquestrants, les agents filmogènes, les agents de traitement du cheveu, les agents dispersants, les agents de conditionnement du cheveu, les agents conservateurs et les agents opacifiants.

17. Composition tinctoriale selon l'une quelconque des revendications 5 à 16, caractérisée par le fait qu'elle a un pH compris entre 4 et 10,5, et de préférence entre 5 et 10.

18. Procédé de teinture des fibres kératiniques, et notamment des cheveux humains par coloration directe, caractérisé par le fait qu'on applique la composition tinctoriale selon l'une quelconque des revendications 5 à 17 sur les fibres kératiniques sèches ou humides, et on sèche ces fibres kératiniques sans rinçage intermédiaire.

19. Procédé de teinture des fibres kératiniques, et notamment des cheveux humains par coloration directe, caractérisé par le fait qu'on applique la composition tinctoriale selon l'une quelconque des revendications 5 à 17 sur les fibres kératiniques sèches ou humides et qu'après avoir laissé agir la composition pendant 3 à 60 minutes, de préférence pendant 5 à 45 minutes, on rince les fibres kératiniques, puis on les sèche.

## Patentansprüche

1. Hydroxypropyliertes 2-Nitro-p-phenylendiamin der Formel: 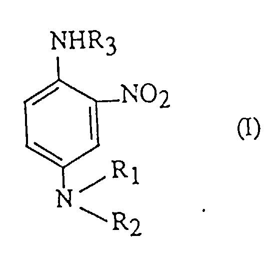 worin
- R₁ einen C₁₋₄-Alkylrest und
- R₂ und R₃, unabhängig voneinander, einen β-Hydroxyethyl-, β-Hydroxypropyl-, γ-Hydroxypropyl- oder einen β,γ-Dihydroxypropylrest darstellen, mit der Maßgabe, daß mindestens einer der Reste R₂ oder R₃ einen γ-Hydroxypropylrest darstellt,
sowie kosmetisch geeignete Salze dieser Verbindung.

2. Verbindung gemäß Anspruch 1,
dadurch **gekennzeichnet**, daß
die durch R₁ dargestellte C₁₋₄-Alkylgruppe ein Methyl-, Ethyl- oder n-Propylrest ist.

3. Verbindung gemäß Anspruch 1 oder 2,
dadurch **gekennzeichnet**, daß
sie aus den folgenden Verbindungen ausgewählt ist:
1-(γ-Hydroxypropyl)amino-4-(N-ethyl,N-β-hydroxyethyl)amino-2-nitrobenzol, 1-(γ-Hydroxypropyl)amino-4-(N-methyl,N-β-hydroxyethyl)amino-2-nitrobenzol, 1-(γ-Hydroxypropyl)amino-4-(N-n-propyl,N-β-hydroxyethyl)amino-2-nitrobenzol, 1-(γ-Hydroxypropyl)amino-4-(N-ethyl,N-y-hydroxypropyl)amino-2-nitrobenzol, 1-(β-Hydroxyethyl)amino-4-(N-ethyl,N-γ-hydroxypropyl)amino-2-nitrobenzol und 1-(β-Hydroxypropyl)amino-4-(N-methyl, N-γ-hydroxypropyl)amino-2-nitrobenzol sowie aus den kosmetisch geeigneten Salzen dieser Verbindungen.

4. Verbindung der Formel: 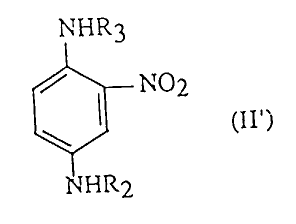 worin der Rest R₂ der γ-Hydroxypropylrest und der Rest R₃ der β-Hydroxyethyl-, β-Hydroxypropyl- oder der γ-Hydroxypropylrest sind, sowie ihre kosmetisch geeigneten Salze.

5. Färbezusammensetzung zur Direktfärbung keratinischer Fasern und insbesondere der menschlichen Haare,
dadurch **gekennzeichnet**, daß
sie in einem wässrigen, alkoholischen oder hydroalkoholischen Trägermittel mindestens eine Verbindung der Formel (I) gemäß einem der Ansprüche 1 bis 3 oder eines ihrer kosmetisch geeigneten Salze enthält.

6. Färbezusammensetzung gemäß Anspruch 5,
dadurch **gekennzeichnet**, daß
sie eine Verbindung der Formel (I) enthält, ausgewählt aus den folgenden Verbindungen:
1-(γ-Hydroxypropyl)amino-4-(N-ethyl,N-β-hydroxyethyl)amino-2-nitrobenzol, 1-(γ-Hydroxypropyl)amino-4-(N-methyl,N-β-hydroxyethyl)amino-2-nitrobenzol, 1-(γ-Hydroxypropyl)amino-4-(N-n-propyl,N-β-hydroxyethyl)amino-2-nitrobenzol, 1-(γ-Hydroxypropyl)amino-4-(N-ethyl,N-γ-hydroxypropyl)amino-2-nitrobenzol, 1-(β-Hydroxyethyl)amino-4-(N-ethyl,N-γ-hydroxypropyl)amino-2-nitrobenzol und 1-(β-Hydroxypropyl)amino-4-(N-methyl, N-γ-hydroxypropyl)amino-2-nitrobenzol sowie aus den kosmetisch geeigneten Salzen dieser Verbindungen.

7. Färbezusammensetzung zur Direktfärbung keratinischer Fasern und insbesondere der menschlichen Haare,
dadurch **gekennzeichnet**, daß
sie in einem wässrigen, alkoholischen oder hydroalkoholischen Trägermittel mindestens eine Verbindung der Formel (II') gemäß Anspruch 4 oder eines ihrer kosmetisch geeigneten Salze enthält.

8. Färbezusammensetzung zur Direktfärbung keratinischer Fasern und insbesondere der menschlichen Haare,
dadurch **gekennzeichnet**, daß
sie in einem wässrigen, alkoholischen oder hydroalkoholischen Trägermittel mindestens eine Verbindung der Formel: 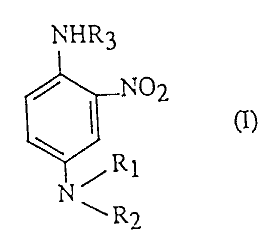 worin:
- R₁ einen C₁₋₄-Alkyl-, β-Hydroxyethyl-, β-Hydroxypropyl- oder einen γ-Hydroxypropylrest und
- R₂ und R₃, unabhängig voneinander, einen β-Hydroxyethyl-, β-Hydroxypropyl-, γ-Hydroxypropyl- oder einen β,γ-Dihydroxypropylrest darstellen, mit der Maßgabe, daß mindestens einer der Reste R₁, R₂ oder R₃ einen γ-Hydroxypropylrest darstellt, und daß die beiden anderen Reste nicht gleichzeitig einen β-Hydroxyethylrest bedeuten, oder eine Verbindung der Formel (II') gemäß Anspruch 4 oder eines von deren kosmetisch geeigneten Salzen, zusammen mit mindestens einem nitrierten benzolischen gelben oder gelb-grünen Farbstoff, ausgewählt aus den folgenden Verbindungen:
1-β-Hydroxyethyloxy-3-methylamino-4-nitrobenzol,
1-(Methylamino)-2-nitro-5-(β,γ-dihydroxypropyl)oxybenzol,
1-(β-Hydroxyethylamino)-2-methoxy-4-nitrobenzol,
1-(β-Aminoethylamino)-2-nitro-5-methoxybenzol,
1,3-Di(β-hydroxyethylamino)-4-nitro-6-chlorbenzol,
1-Amino-2-nitro-6-methylbenzol,
1-(β-Hydroxyethylamino)-2-hydroxy-4-nitrobenzol,
N-(β-Hydroxyethyl)-2-nitro-3-trifluormethylanilin,
4-β-Hydroxyethylamino-3-nitrobenzolsulfonsäure,
4-Ethylamino-3-nitrobenzoesäure,
4-(β-Hydroxyethyl)amino-3-nitrochlorbenzol,
4-(β-Hydroxyethyl)amino-3-nitromethylbenzol,
4-(β,γ-Dihydroxypropyl)amino-3-nitromethylbenzol,
4-(β,γ-Dihydroxypropyl)amino-3-nitrotrifluormethylbenzol,
1-β-Ureidoethylamino-4-nitrobenzol,
O,N-Bis(β-hydroxyethyl)-2-amino-5-nitrophenol,
1,3-Diamino-4-nitrobenzol,
l-Hydroxy-2-amino-5-nitrobenzol,
l-Amino-2-[tris(hydroxymethyl)methyl]amino-5-nitrobenzol,
1-(β-Hydroxyethyl)amino-2-nitrobenzol und aus
4-(β-Hydroxyethylamino-3-nitrobenzamid,
enthält.

9. Färbezusammensetzung gemäß einem der Ansprüche 5 bis 8,
dadurch **gekennzeichnet**, daß sie nitrierte benzolische rote Farbstoffe enthält, ausgewählt aus den folgenden Verbindungen:
1-Hydroxy-3-nitro-4-(γ-hydroxypropylamino)benzol,
N-(β-Hydroxyethyl)amino-3-nitro-4-aminobenzol,
1-Amino-3-methyl-4-(β-hydroxyethyl)amino-6-nitrobenzol,
1-Hydroxy-3-nitro-4-N-β-hydroxyethylaminobenzol,
1,4-Diamino-2-nitrobenzol,
1-Amino-2-nitro-4-methylaminobenzol,
N-(β-Hydroxyethyl)-2-nitro-p-phenylendiamin,
1-Amino-2-nitro-4-(β-hydroxyethylamino)-5-chlorbenzol,
2-Nitro-4-aminodiphenylamin und aus
1-Amino-3-nitro-6-hydroxybenzol.

10. Färbezusammensetzung gemäß einem der Ansprüche 5 bis 8,
dadurch **gekennzeichnet**, daß
sie nitrierte benzolische orangefarbene Farbstoffe enthält, ausgewählt aus den folgenden Verbindungen:
1-(β-Aminoethyl)amino-2-nitro-4-(β-hydroxyethyl)oxybenzol,
1-(β,γ-Dihydroxypropyl)oxy-3-nitro-4-(β-hydroxyethyl)aminobenzol,
1-Hydroxy-3-nitro-4-aminobenzol,
1-Hydroxy-2-amino-4,6-dinitrobenzol,
1-Methoxy-3-nitro-4-(β-hydroxyethylamino)benzol,
2-Nitro-4'-hydroxydiphenylamin und aus
1-Amino-2-nitro-4-hydroxy-5-methylbenzol.

11. Färbezusammensetzung gemäß einem der Ansprüche 5 bis 10,
dadurch **gekennzeichnet**, daß
sie weitere Direktfarbstoffe enthält, ausgewählt aus Azo-, Anthrachinon-Farbstoffen, Triarylmethanderivaten und aus basischen Farbstoffen.

12. Färbezusammensetzung gemäß einem der Ansprüche 5 bis 11,
dadurch **gekennzeichnet**, daß
sie 0,01 bis 10 und vorzugsweise 0,1 bis 5 Gew.%, ausgedrückt als freie Base, Verbindung der Formel (I) oder (II') enthält.

13. Färbezusammenseztung gemäß Anspruch 8,
dadurch **gekennzeichnet**, daß
sie 0,05 bis 3 Gew.% nitrierte benzolische gelbe oder gelbgrüne Farbstoffe enthält.

14. Färbezusammensetzung gemäß einem der Ansprüche 5 bis 13,
dadurch **gekennzeichnet**, daß
sie ausserdem 0,05 bis 10 Gew.% weitere Direktfarbstoffe enthält.

15. Färbezusammensetzung gemäß einem der Ansprüche 5 bis 14,
dadurch **gekennzeichnet**, daß
sie organische Lösungsmittel, ausgewählt aus Alkoholen, Glycolen und aus Glycolethern, in Konzentrationen von 0,5 bis 20 und vorzugsweise von 2 bis 10 Gew.% enthält, bezogen auf das Gesamtgewicht der Zusammensetzung.

16. Färbezusammensetzung gemäß einem der Ansprüche 5 bis 15,
dadurch **gekennzeichnet**, daß
sie mindestens einen Hilfsstoff enthält, ausgewählt aus Fettamiden in Konzentrationen von 0,05 bis 10 Gew.%, aus anionischen, kationischen, nicht-ionischen und amphoteren oberflächenaktiven Mitteln oder aus deren Mischungen, in Konzentrationen von 0,1 bis 50 Gew.%, aus Verdickungsmitteln in Konzentrationen von 0,2 bis 5 Gew.%, aus Antioxidantien, Parfüm-Produkten, Beaufschlagungsmitteln, filmbildenden Mitteln, Behandlungsmitteln für das Haar, Dispergiermitteln, Konditioniermitteln für das Haar, Konservierungsmitteln und aus opak machenden Mitteln.

17. Färbezusammensetzung gemäß einem der Ansprüche 5 bis 16,
dadurch **gekennzeichnet**, daß
sie einen pH-Wert von 4 bis 10,5 und vorzugsweise von 5 bis 10 aufweist.

18. Verfahren zur Färbung keratinischer Fasern und insbesondere der menschlichen Haare durch Direkt färbung,
dadurch **gekennzeichnet**, daß
man die Färbezusammensetzung gemäß einem der Ansprüche 5 bis 17 auf die keratinischen trockenen oder feuchten Fasern aufbringt und diese keratinischen Fasern ohne Zwischenspülung trocknet.

19. Verfahren zur Färbung keratinischer Fasern und insbesondere der menschlichen Haare durch Direktfärbung,
dadurch **gekennzeichnet**, daß
man die Färbezusammensetzung gemäß einem der Ansprüche 5 bis 17 auf die keratinischen trockenen oder feuchten Fasern aufbringt und nach Einwirkung der Zusammensetzung über 3 bis 60 und vorzugsweise 5 bis 45 Minuten die keratinischen Fasern spült und sie dann trocknet.

## Claims

1. Hydroxypropylated 2-nitro-p-phenylenediamine having the formula: 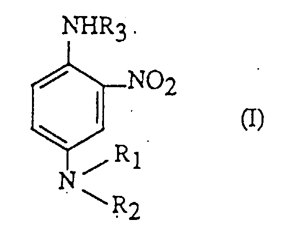 in which :
- R₁ represents a C₁-C₄ alkyl radical,
- R₂ and R₃, independently of one another, represent a β-hydroxyethyl, β-hydroxypropyl, γ-hydroxypropyl or β,γ-dihydroxypropyl radical, with the proviso that at least one of the radicals R₂ or R₃ represents a γ-hydroxypropyl radical,
and cosmetically acceptable salts thereof.

2. Compound according to Claim 1, characterized in that the C₁-C₄ alkyl group represented by R₁ is a methyl, ethyl or n-propyl radical.

3. Compound according to Claim 1 or 2, characterized in that it is chosen from the following compounds : 1-(γ-hydroxypropyl)amino-4-(N-ethyl-N-β-hydroxyethyl)-amino-2-nitrobenzene, 1-(γ-hydroxypropyl)amino-4-(N-methyl-N-β-hydroxyethyl)amino-2-nitrobenzene, 1-(γ-hydroxypropyl)amino-4-(N-n-propyl-N-β-hydroxyethyl)amino-2-nitrobenzene, 1-(γ-hydroxypropyl)amino-4-(N-ethyl-N-γ-hydroxypropyl)amino-2-nitrobenzene, 1-(β-hydroxyethylamino-4-(N-ethyl-N-γ-hydroxypropyl)amino-2-nitrobenzene [sic] and 1-(β-hydroxypropyl)amino-4-(N-methyl-N-γ-hydroxypropyl)amino-2-nitrobenzene and the cosmetically acceptable salts thereof.

4. Compound of formula : 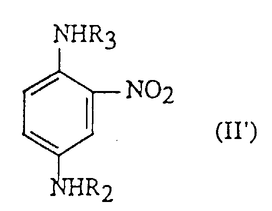 in which the radical R₂ is the γ-hydroxypropyl radical and the radical R₃ is the β-hydroxyethyl, β-hydroxypropyl or γ-hydroxypropyl radical, and the cosmetically acceptable salts thereof.

5. Dye composition for the direct dyeing of keratinous fibres, and in particular human hair, characterized in that it contains, in an aqueous, alcoholic or aqueous-alcoholic vehicle, at least one compound of the formula (I) according to any one of Claims 1 to 3 or one of the cosmetically acceptable salts thereof.

6. Dye composition according to Claim 5, characterized in that it contains a compound of formula (I) chosen from the following compounds: 1-(γ-hydroxypropyl)-amino-4-(N-ethyl-N-β-hydroxyethyl)amino-2-nitrobenzene, 1-(γ-hydroxypropyl)amino-4-(N-methyl-N-β-hydroxyethyl)-amino-2-nitrobenzene, 1-(γ-hydroxypropyl)amino-4-(N-n-propyl-N-β-hydroxyethyl)amino-2-nitrobenzene, 1-(γ-hydroxypropyl)amino-4-(N-ethyl-N-γ-hydroxypropyl)amino-2-nitrobenzene, 1-(β-hydroxyethylamino-4-(N-ethyl-N-γ-hydroxypropyl)amino-2-nitrobenzene [sic] and 1-(β-hydroxypropyl)amino-4-(N-methyl-N-γ-hydroxypropyl)amino-2-nitrobenzene and the cosmetically acceptable salts thereof.

7. Dye composition for the direct dyeing of keratinous fibres and in particular human hair, characterized in that it contains, in an aqueous, alcoholic or aqueous-alcoholic vehicle, at least one compound of formula (II') according to Claim 4 or one of the cosmetically acceptable salts thereof.

8. Dye composition for the direct dyeing of keratinous fibres and in particular human hair, characterized in that it contains, in an aqueous, alcoholic or aqueous-alcoholic vehicle, at least one compound of formula: 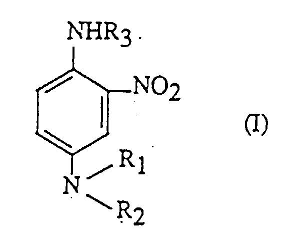 in which :
- R₁ represents a C₁-C₄ alkyl, β-hydroxyethyl, β-hydroxypropyl or γ-hydroxypropyl radical,
- R₂ and R₃, independently of one another, represent a β-hydroxyethyl, β-hydroxypropyl, γ-hydroxypropyl or β,γ-dihydroxypropyl radical, with the proviso that at least one of the radicals R₁, R₂ or R₃ represents a γ-hydroxypropyl radical and that the other two radicals cannot simultaneously denote a β-hydroxyethyl radical, or (II') according to Claim 4, or one of the cosmetically acceptable salts thereof, in combination with at least one yellow or green-yellow nitrobenzene dye chosen from the following compounds: 1-β-hydroxyethyloxy-3-methylamino-4-nitrobenzene, 1-(methylamino)-2-nitro-5-(β,γ-dihydroxypropyl)oxybenzene, 1-(β-hydroxyethylamino)-2-methoxy-4-nitrobenzene, 1-(β-aminoethylamino)-2-nitro-5-methoxybenzene, 1,3-di(β-hydroxyethylamino)-4-nitro-6-chlorobenzene, 1-amino-2-nitro-6-methylbenzene, 1-(β-hydroxyethylamino)-2-hydroxy-4-nitrobenzene, N-(β-hydroxyethyl)-2-nitro-4-trifluoromethylaniline, 4-β-hydroxyethylamino-3-nitrobenzenesulphonic acid, 4-ethylamino-3-nitrobenzoic acid, 4-(β-hydroxyethyl)amino-3-nitrochlorobenzene, 4-(β-hydroxyethyl)amino-3-nitromethylbenzene, 4-(β,γ-dihydroxypropyl)amino-3-nitrotrifluoromethylbenzene, 1-β-ureidoethylamino-4-nitrobenzene, O,N-bis(β-hydroxyethyl)-2-amino-5-nitrophenol, [lacuna] 1-hydroxy-2-amino-5-nitrobenzene, 1-amino-2-[tris-(hydroxymethyl)methyl] amino-5-nitrobenzene, 1-(β-hydroxyethyl)amino-2-nitrobenzene and 4-(β-hydroxyethylamino)-3-nitrobenzamide.

9. Dye composition according to any one of Claims 5 to 8, characterized in that it contains red nitrobenzene dyes chosen from the following compounds: 1-hydroxy-3-nitro-4-(γ-hydroxypropylamino)benzene, N-(β-hydroxyethyl)amino-3-nitro-4-aminobenzene [sic], 1-amino-3-methyl-4-(β-hydroxyethyl)amino-6-nitrobenzene, 1-hydroxy-3-nitro-4-N-(β-hydroxyethyl)aminobenzene, 1,4-diamino-2-nitrobenzene, 1-amino-2-nitro-4-methylaminobenzene, N-(β-hydroxyethyl)-2-nitro-para-phenylenediamine, 1-amino-2-nitro-4-(β-hydroxyethylamino)-5-chlorobenzene, 2-nitro-4-aminodiphenylamine and 1-amino-3-nitro-6-hydroxybenzene.

10. Dye composition according to any one of Claims 5 to 8, characterized in that it contains orange nitrobenzene dyes chosen from the following compounds: 1-(β-aminoethyl)amino-2-nitro-4-(β-hydroxyethyl)oxybenzene, 1-(β,γ-dihydroxypropyl)oxy-3-nitro-4-(β-hydroxyethyl)-aminobenzene, 1-hydroxy-3-nitro-4-aminobenzene, 1-hydroxy-2-amino-4,6-dinitrobenzene, 1-methoxy-3-nitro-4-(β-hydroxyethylamino)benzene, 2-nitro-4'-hydroxydiphenylamine and 1-amino-2-nitro-4-hydroxy-5-methylbenzene.

11. Dye composition according to any one of Claims 5 to 10, characterized in that it contains other direct dyes chosen from azo dyes, anthroquinone dyes, triarylmethane derivatives and basic dyes.

12. Dye composition according to any one of Claims 5 to 11, characterized in that it contains 0.01 to 10 % by weight, and preferably 0.1 to 5 % by weight, expressed as free base, of a compound of formula (I) or (II').

13. Dye composition according to Claim 8, characterized in that it contains 0.05 to 3 % by weight of yellow or green-yellow nitrobenzene dyes.

14. Dye composition according to any one of Claims 5 to 13, characterized in that it additionally contains 0.05 to 10 % by weight of other direct dyes.

15. Dye composition according to any one of Claims 5 to 14, characterized in that it contains organic solvents chosen from alcohols, glycols and glycol ethers, in concentrations of between 0.5 and 20 % by weight, and preferably of between 2 and 10 % by weight, relative to the total weight of the composition.

16. Dye composition according to any one of Claims 5 to 15, characterized in that it contains at least one adjuvant chosen from fatty amides in concentrations of between 0.05 and 10 % by weight, anionic, cationic, nonionic or amphoteric surface-active agents, or mixtures thereof, in concentrations of between 0.1 and 50 % by weight, thickening agents in concentrations of between 0.2 and 5 % by weight, antioxidants, fragrances, sequestering agents, film-forming agents, hair-treatment agents, dispersing agents, hair-conditioning agents, preserving agents and opacifying agents.

17. Dye composition according to any one of Claims 5 to 16, characterized in that it has a pH of between 4 and 10.5 and preferably between 5 and 10.

18. Process for dyeing keratinous fibres, and in particular human hair, by direct dyeing, characterized in that the dye composition according to any one of Claims 5 to 17 is applied to the dry or wet keratinous fibres, and these keratinous fibres are dried without intermediate rinsing.

19. Process for dyeing keratinous fibres, and in particular human hair, by direct dyeing, characterized in that the dye composition according to any one of Claims 5 to 17 is applied to the dry or wet keratinous fibres, and in that, after leaving the composition to act for 3 to 60 minutes, preferably for 5 to 45 minutes, the keratinous fibres are rinsed and then dried.
